# EUROPEAN PATENT APPLICATION

(11) **EP 3 800 254 A1**
(43) Date of publication of application: **07.04.2021**
(21) Application number: 20189217.1
(22) Date of filing: 07.06.2013
(51) Int. Cl.: C12N 15/11, C12N 15/67

(54) **PULMONARY DELIVERY OF MESSENGER RNA**

(30) Priority: 08.06.2012 US 201261657344 P
(62) Divisional of application: 13735212.6
(71) Applicant: Ethris GmbH, 82152 Planegg (DE)
(72) Inventor: GEIGER, Johannes, 81249 Munich (DE); ANEJA, Manish Kumar, 81373 Munich (DE); RUDOLPH, Carsten, 85152 Krailling (DE)
(74) Representative: Vossius & Partner Patentanwälte Rechtsanwälte mbB

(57) **Abstract**

The invention discloses a method for expressing an mRNA in lung wherein
- the mRNA to be expressed is combined with polyethyleneimine (PEI) to provide a combination comprising the mRNA and PEI;
- the combination comprising the mRNA and PEI is administered to lung where it enters lung cells; and
- the mRNA is expressed in the lung cells.

## Description

The present invention relates to a method for expressing mRNA in lung.

Messenger RNAs (mRNA) are polymers which are built up of nucleoside phosphate building blocks mainly with adenosine, cytidine, uridine and guanosine as nucleosides, which as intermediate carriers bring the genetic information from the DNA in the cell nucleus into the cytoplasm, where it is translated into proteins. They are thus suitable as alternatives for gene expression.

The elucidation of the biochemical processes in the cell and the elucidation of the human genome have revealed connections between deficient genes and diseases. Hence there has long been the desire to heal diseases due to deficient genes by gene therapy. The expectations were high, but initial attempts at this failed and only recently progress has been reported. A first approach to gene therapy consisted in bringing the intact DNA of a deficient or defective gene into the cell nucleus in a vector in order to achieve the expression of the intact gene and thus the provision of the missing or defective protein. These attempts were for the most part not successful and the less successful attempts were burdened with substantial side effects, in particular elevated tumorigenesis. Only very recently more promising results were reported which, however, are still far away from being approved.

Furthermore, there are diseases which are due to a lack of proteins or a protein defect, without this being attributable to a genetic defect. In such a case also, consideration is being given to producing the relevant proteins in vivo by administration of DNA. The provision of factors which play a part in the metabolism and are destroyed or inhibited for pathological or non-pathological reasons could also be effected by a zero or low side effect nucleic acid therapy.

The use has also already been proposed of mRNAs for the therapy of hereditary diseases in order to treat gene defects which lead to diseases. The advantage in this is that the mRNA only has to be introduced into the cytoplasm of a cell, but does not have to be translocated into the nucleus. Translocation into the nucleus is difficult and inefficient; moreover there is a considerable risk of the chromosomal DNA being altered if the vector or parts thereof become incorporated into the genome.

Admittedly it could be shown that in vitro transcribed messenger RNA can in fact be expressed in mammalian tissue, however further hurdles arose in the attempt to use mRNA for the therapy of diseases. The lack of stability of the mRNA had the effect that the desired protein could not be made available in sufficient quantity in the mammalian tissue. A further substantial disadvantage resulted from the fact that mRNA triggers considerable immunological reactions. It is presumed that these strong immune reactions arise through binding to Toll-like receptors such as TLR3, TLR7, TLR8 and helicase RIG-1.

In order to prevent an immunological reaction, it was proposed in WO 2007/024708 A to use RNA wherein one of the four ribonucleotides is replaced by a modified nucleotide. In particular, it was investigated how mRNA behaves when the uridine is totally replaced by pseudouridine. It was found that such an RNA molecule is significantly less immunogenic. Furthermore, it was further proposed to use RNA with a sequence which encodes a protein or protein fragment, wherein the RNA contains a combination of unmodified and modified nucleotides, wherein 5 to 50% of the uridine nucleotides and 5 to 50% of the cytidine nucleotides are respectively modified uridine nucleotides and modified cytidine nucleotides. It was found that such an RNA molecule is significantly less immunogenic and even more stable. It was further proposed that such RNA can be used to prevent death in mice suffering from surfactant protein B (SP-B) deficiency by repeated intratracheal aerosol application in mice. These observations thus demonstrate the promise of such RNA to treat a life-threatening inherited and acquired pulmonary diseases and addresses a high unmet medical need. However, with respect to application in patients this application procedure was not yet suitable for repeated aerosol application because of required anaesthesia and RNA degradation using standard clinically used nebulizers.

In order to be able to provide the lung of the body with necessary or beneficial proteins and/or to treat a disease due to missing or deficient proteins with RNA, it is desirable to have a method for repeated aerosol application available which avoids repeated anaesthesia of the patient. At the same time, however, this method must not cause a decrease in RNA efficacy to a significant extent.

It was proposed in EP 1 173 224 B1 to use polyethylenimine (PEI) 25 kDa/DNA formulations for aerosol delivery of genes to the lung suitable to overcome the previously unresolved problem of repeated anaesthesia and a marked decrease in efficiency that accompanies the process of nebulisation in comparison to in vitro transfection. It was found that PEI/DNA formulations are resistant to jet-nebulizer-induced reduction of transfection efficiency and are superior to previously optimized lipid-based formulations when delivered in vivo by jet-nebulizer. Specifically, EP 1 173 224 B1 discloses a method of targeting therapy such as gene therapy via the respiratory tract, comprising the step of delivering aqueous dispersions of a genetic macromolecule complexed with polyethyleneimine via small particle aerosol via a respiratory tract of an individual. Representative examples of genetic macromolecules according to the methods of the invention included DNA, RNA and other nucleic acid species. However, the invention has been reduced to practice in the examples comprised in the patent for DNA delivery only but not for mRNA. Furthermore, complexes of plasmid DNA, encoding a gene of interest, with PEI were formed by mixing plasmid DNA dissolved in water with appropriate amounts of PEI dissolved in PBS. However, Rudolph et al. (Mol Ther. 2005, 12: 493-501) demonstrated that PEI-DNA complexes when assembled and nebulized in hypoosmotic distilled water yielded 57- and 185-fold higher expression levels in mouse lungs than those in isotonic 5% glucose or Hepes-buffered saline, respectively. Surprisingly PEI-DNA complexes when assembled and nebulized in PBS were entirely ineffective. This was primarily attributed to the fact that PEI gene vectors formulated in PBS resulted in large diameters (848±142 nm) which were kinetically instable and led to precipitation. It was also found that aerosolized nanogram quantities of pDNA 350 ng) complexed to PEI (yielded transfection levels 15-fold higher than a 140-fold higher dose (50 µg) of the same vector applied directly to the lungs of mice via intratracheal intubation.

Furthermore, Bettinger et al. (Nucleic Acids Res. 2001, 29: 3882-91) demonstrated that lipoplexes, but not polyplexes based on polyethyleneimine (branched PEI 25 and linear PEI 22 kDa), poly(L-lysine) (PLL, 54 kDa) or dendrimers, mediated efficient translation of mRNA in transfected B16-F10 cells. Lack of expression with PEI 25 kDa/mRNA or PLL 54 kDa/mRNA in a cell-free translation assay and following cytoplasmic injection into Rat1 cells indicated that these polyplexes were too stable to release mRNA. It was demonstrated that the strength of electrostatic interaction between the mRNA and the transfection agent had a dramatic effect on the level of expression achieved, with thermodynamically stable polyplex vectors, e.g. PEI-mRNA, being less suitable for mRNA translation. Decreasing the electrostatic interaction between the carrier and the mRNA by using shorter polycations gave major increases in expression, with mRNA polyplexes formed using low molecular weight PEI and PLL achieving 5-fold greater levels of luciferase expression than DOTAP/mRNA. However, the polyplexes formed using low molecular weight polycations lost their endosomolytic activity and required chloroquine to mediate mRNA expression. Endosomolysis was restored by conjugating low molecular weight PEI to the membrane-active peptide melittin, and high levels of mRNA expression were demonstrated in the absence of chloroquine. Together these observations demonstrate that single stranded mRNA binding to cationic polymers is largely stronger than pDNA binding. This has further been confirmed by Huth et al. (J. Gene Med. 2006, 8: 1416-1424) who suggested that cytosolic RNA is involved in pDNA release from cationic polymers as a precondition for nuclear entry, transcription and successful transgene expression. In conclusion these observations suggested that PEI 25 kDa is incapable of mediating functional mRNA delivery into cells.

As a continuously repeated treatment involving intubation is not compatible with quality-of-life requirements, the task underlying the present invention was providing a compliant and non-invasive method for pulmonary delivery of messenger RNA resulting in pulmonary expression of a protein encoded by said mRNA.

The prior art is deficient in non-invasive methods for pulmonary delivery of mRNA.

Hence an object of the present invention is to provide a method for delivery of an mRNA therapeutic agent through non-invasive pulmonary application that results in the production of effective levels of encoded protein inside the lung.

Therefore, the present invention provides a method for expressing an mRNA in lung wherein
- the mRNA to be expressed is combined with polyethyleneimine (PEI) to provide a combination comprising the mRNA and PEI;
- the combination comprising the mRNA and PEI is administered to lung where it enters lung cells; and
- the mRNA is expressed in the lung cells.

The present invention is specifically suitable for use in human medicine. Therefore, the lung to be treated according to the present invention is preferably the lung of a human patient, preferably a human patient with a pulmonary defect, especially a pulmonary defect selected from the group surfactant protein B (SPB) deficiency, ATP-binding cassette sub-family A member 3 (ABCA3) deficiency, cystic fibrosis, alpha-1 antitrypsin (A1AT) deficiency, lung cancer, surfactant protein C (SPC) deficiency, alveolar proteinosis, sarcoidosis, acute and chronic bronchitis, emphysema, McLeod-Syndrom, chronic obstructive pulmonary disease (COPD), asthma bronchiale, bronchiectasis, pneumoconiosis, asbestosis, Acute Respiratory Distress Syndrome (ARDS), Infant respiratory distress syndrome (IRDS), pulmonary oedema, pulmonary eosinophilia, Löffler's pneumonia, Hamman-Rich syndrome, idiopathic pulmonary fibrosis, interstitial pulmonary diseases, primary ciliary dyskinesia, pulmonary arterial hypertension (PAH) and STAT5b deficiency.

Furthermore, the lung of the human patient may serve as a bioreactor for secretion of proteins expressed by the mRNA from the lung cells into the blood circulation such as erythropoietin, clotting defects such as hemophilia A and B, complement defects such as protein C deficiency, thrombotic thrombocytopenic purpura (TTP, ADAMTS 13 deficiency) and congenital hemochromatoses (e.g. Hepcidin deficiency).

Expression of an mRNA from lung cells can also be used for vaccination against pulmonary infectious diseases such respiratory syncytial virus (RSV) infection, parainfluenza virus (PIV) infection, influenza virus infection, rhinoviruses infection, and severe acute respiratory syndrome (corona virus (SARS-CoV) infection, tuberculosis, Pseudomonas aeruginosa infection, Burkholderia cepacia infection, Methicillin-Resistant Staphylococcus aureus (MRSA) infection, and Haemophilus influenzae infection. For such vaccination purposes, the mRNA delivered encodes one or more antigens of the pathogen.

The PEI to be administered in the course of the present invention is usually not critical (Morimoto et al., Mol. Ther. 7 (2003), 254-261), however, it is advantageous within the course of the present invention to use a PEI which has a molecular weight of 1 kDa to 1000 kDa, preferably from 10 kDa to 50 kDa, especially from 20 to 30 kDa.

According to a preferred embodiment of the present invention
the PEI comprises a targeting ligand, preferably a IP₁ receptor ligand, more preferred a prostacyclin analogue, especially Iloprost (5-{ (E)-(1S,5S,6R,7R)-7-hydroxy-6[(E)-(3S, 4RS)-3-hydroxy-4-methyl-1-octen-6-inyl]-bi-cyclo[3.3.0]octan-3-ylidene}pentanoic acid) or Treprostinil ((1R,2R,3aS,9aS)-[[2,3,3a,4,9,9a-hexahydro-2-hydroxy-1-[(3S)-3-hydroxyoctyl]-1H-benz[f]inden-5-yl] oxy]acetic acid); or β₂-aderonceptor ligands, especially Clenbuterol (Elfinger et al., J. Control. Release 2009, 135: 234-241), Lactoferrin (Elfinger et al., Biomaterials 2007, 28: 3448-3455), uronic acids (Weiss et al., Biomaterials 2006, 27: 2302-2312), or lectins (Bies et al., Adv. Drug Deliv. Rev. 2004, 56: 425-435).

The use of IP₁ receptor ligand, more preferred a prostacyclin analogue, for target-specific delivering PEI-based pharmaceutical formulation to lung cells, especially to bronchial or alveolar epithelial cells has been disclosed and made available by WO 2011/076391 A.

With the method according to the present invention, it is preferred to deliver an mRNA to lung cells which has medical benefit, especially mRNA which replaces, overturns, antagonises or suppresses a gene which has pathogenic effect in the lung or for this patient. It is specifically preferred to deliver an mRNA which substitutes for a defect gene in that lung cell. Accordingly, preferred embodiments of the present invention are methods and compositions wherein the mRNA encodes Cystic fibrosis transmembrane conductance regulator (CFTR), Surfactant Protein B (SPB), ATP-binding cassette sub-family A member 3 (ABCA3) or alpha-1 antitrypsin (A1AT), surfactant protein C (SPC), granulocyte macrophage colony stimulating factor, erythropoietin, Factor VIII, Factor IX, van Willebrand Factor, ADAMTS 13, Hepcidin, angiotensin converting enzyme II or antigens of viral and bacterial pathogens.

The present invention also relates to a pharmaceutical composition comprising mRNA and PEI for use in a method for expressing the mRNA in lung. The pharmaceutical composition according to the present invention is specifically suited for the treatment of a pulmonary defect, especially a pulmonary defect selected from the group surfactant protein B (SPB) deficiency, ATP-binding cassette sub-family A member 3 (ABCA3) deficiency, cystic fibrosis, alpha-1 antitrypsin (A1AT) deficiency; lung cancer, surfactant protein C (SPC) deficiency, alveolar proteinosis, sarcoidosis, acute and chronic bronchitis, emphysema, McLeod-Syndrom, chronic obstructive pulmonary disease (COPD), asthma bronchiale, bronchiectasis, pneumoconiosis, asbestosis, Acute Respiratory Distress Syndrome (ARDS), Infant respiratory distress syndrome (IRDS), pulmonary oedema, pulmonary eosinophilia, Löffler's pneumonia, Hamman-Rich syndrome, idiopathic pulmonary fibrosis, interstitial pulmonary diseases, primary ciliary dyskinesia, pulmonary arterial hypertension (PAH) and STAT5b deficiency, clotting defects, especially hemophilia A and B; complement defects, especially protein C deficiency, thrombotic thrombocytopenic purpura and congenital hemochromatosis, especially Hepcidin deficiency; pulmonary infectious diseases, preferably respiratory syncytial virus (RSV) infection, parainfluenza virus (PIV) infection, influenza virus infection, rhinoviruses infection, and severe acute respiratory syndrome (corona virus (SARS-CoV) infection, tuberculosis, Pseudomonas aeruginosa infection, Burkholderia cepacia infection, Methicillin-Resistant Staphylococcus aureus (MRSA) infection, and Haemophilus influenzae infection.

A preferred pharmaceutical composition according to the present invention further comprises at least one fluorocarbon. Aerosol treatment with perfluorocarbons generally shows improved gas exchange and reduced pulmonary inflammatory reaction independently from molecular structure and vapor pressure of the perfluorocarbons. Although differences in vapor pressure and molecular structure may account for varying optimal dosing strategies, several different perfluorocarbons were shown to be principally suitable for aerosol treatment, for example, perfluorocycloether (FC77), perfluorooctylbromide, or perfluorotributylamine (FC43).

Accordingly, the present invention is preferably provided as
an aerosol. In a preferred embodiment, the pharmaceutical composition according to the present invention intended for pulmonary administration is combined with perfluorocarbon, which is administered previously or simultaneously with the pharmaceutical composition in order to increase the transfection efficiency.

In a preferred embodiment, the mRNA/PEI combination according to the invention is provided in a form suitable for uptake via the lung, e.g. by inhalation. Suitable formulae for this are known to those skilled in the art. In this case the preparation is in a form which can be introduced into the respiratory tract via normal nebulizers or inhalers, e.g. as a liquid for nebulizing or as a powder. Devices for administration as liquid are known, and ultrasound nebulizers or nebulizers with a perforated oscillating membrane which operate with low shear forces compared to nozzle jet nebulizers are suitable. Also suitable are powder aerosols. mRNA complexed with PEI is available after the freeze-drying with the sugar sucrose as powder that can then be crushed to a respirable size and moreover shows biological activity.

Preferably the mRNA/PEI combination is administered intratracheally as an aerosol by spraying at high pressure.

In a specifically preferred embodiment of the present invention, the pharmaceutical preparation is provided as an aerosol containing magnetic particles, especially preparations wherein the aerosol contains magnetic particles have a diameter of at least 5 nm and at most 800 nm together with the mRNA/PEI combination (EP 1 924 244 A). The magnetic particles usually have a diameter of at least 50 nm and at most 750 nm, preferably of at least 100 nm and at most 700 nm, more preferably of at least 150 nm and at most 600 nm, still more preferably of at least 200 nm and at most 500 nm, particularly preferably of at least 250 nm and at most 450 nm, most preferably of at least 300 nm and at most 400 nm.

According to a preferred embodiment, the PEI is coupled to the magnetic particles in the aerosol.

Preferably, the aerosol containing magnetic particles according to the present invention consist of metals and/or oxides and/or hydroxides thereof or contain these. According to a preferred embodiment, the magnetic particles consist of metals, or contain these, and are selected from the group consisting of iron, cobalt or nickel, magnetic iron oxides or hydroxides, such as Fe₃O₄, gamma-Fe₂O₃, double oxides or hydroxides of di- or trivalent iron ions with other di- or trivalent metal ions, such as Co²⁺, Mn²⁺, Cu²⁺, Ni²⁺, Cr³⁺, Gd³⁺, Dy³⁺ or Sm³⁺, and any mixtures thereof.

In applying such aerosols containing magnetic particles, the aerosol containing magnetic particles can be deposited by a magnetic field onto the surface of the region of the respiratory tract and/or lung to be treated. Preferably, the magnetic field has a field strength of at least 100 mT (millitesla), at least 200 mT, at least 500 mT or at least 1 T (tesla). Preferably, the magnetic field has a magnetic field gradient of greater than 1 T/m or greater than 10 T/m. According to a preferred embodiment of this method, the magnetic field is a pulsating, an oscillating or pulsating-oscillating magnetic field. Preferably, the magnetic field is matched dynamically to the breathing of the patient and is active only during the resting pauses between in- and exhalation of ex- and inhalation (EP 1 924 244 A).

Particularly suitable is PEI 25 kDa which is used to formulate mRNA which encodes a protein or protein fragment and wherein the formulation is generated in distilled water and which is applied as an aerosol to the lung using a jet-nebulizer.

Specifically preferred embodiments of the present invention are prepared by using aqueous buffers and solvents with low pH and low conductivity. PBS buffer has a pH of 7.4 and a conductivity of 16,500 ± 500 µS/cm (at 25°C). The mRNA in the compositions according to the present invention surprisingly shows increasing stability if solutions of lower conductivity and/or lower pH are applied. For example, autoclaved ultrapure water has a conductivity of 1 ± 0.2 µS/cm (the US Pharmacopeia requires an upper limit for conductivity at 25°C of 1.3) and a pH of 5.0 to 7.0. Tap water filtered through a 0.2-µm filter has a conductivity of 300 ± 5 µS/cm. In preferred embodiments of the present invention, aqueous buffers and solvents with lower pH and lower conductivity than PBS buffer are applied. The pharmaceutical composition according to the present invention has therefore preferably a pH of under 6.5, preferably of 3 to 6, especially of 4 to 5.5 and/or a 25°C conductivity (i.e. a conductivity at 25°C) of 10000 µS/cm or lower, preferably of 1000 or lower, especially of 100 or lower. For example, a specifically preferred embodiment contains pharmaceutically acceptable water (Water for Injection), as defined in the US Pharmacopeia (instead of a buffer, such as PBS).

Of course, the pharmaceutical preparation according to the present invention can further contain pharmaceutically acceptable carriers and/or further auxiliary compounds, especially compounds usually provided in aerosol compositions to be delivered to human lungs.

Replication-deficient viruses have been used most successfully in the field of gene therapy because of their high transfection efficiency. However, the risk of insertional mutagenesis and induction of unwanted immune responses remains still critical for their safe application. On the other hand, nonviral vectors have been intensively investigated for plasmid DNA (pDNA) delivery as a safer alternative although their gene transfer efficiency is still many folds lower than for viral vectors, which has been predominately attributed to the insufficient transport of pDNA into the nucleus. Instead of pDNA, messenger RNA (mRNA) has recently emerged as an attractive and promising alternative in the nonviral gene delivery field. This strategy combines several advantages compared to pDNA: i) the nuclear membrane, which is a major obstacle for pDNA, can be avoided because mRNA exerts its function in the cytoplasm; ii) the risk of insertional mutagenesis can be excluded; iii) the determination and use of an efficient promoter is omitted; iv) repeated application is possible; v) mRNA is also effective in non-dividing cells, and vi) vector-induced immunogenicity may be avoidable.

Gene transfer vehicles based on mRNA have emerged as attractive alternatives to vehicles made of DNA for the potential treatment of genetic disorders or (anti-tumor) vaccination. Its successful application has been demonstrated in cancer immunotherapy, not only because it is possible to deliver all epitopes of entire antigens in one step together but manipulation as well as purification are rather simple, too. In addition, this strategy has several advantages in terms of pharmaceutical safety because mRNA does not integrate into the genome and the transfection remains transient. mRNA encoding versatile antigens combined with delivery to dendritic cells (DCs) is a strong and promising approach to induce immune response in cancer patients.

Thus far, plasmid DNA (pDNA) has been largely used for nonviral gene transfer. However, it can hardly be transfected into non-dividing mammalian cells and, besides, bacterial unmethylated DNA CpG motifs induce strong immune response through Toll-like receptor 9 (TLR9). For example, only 1-10% of DCs are transfected by means of electroporation, cationic polymers or cationic lipids. On the other hand, transfection efficiencies by electroporation of mRNA has been previously shown to reach up to 95% transfected cells. These observations suggest that mRNA transfer is much more effective compared to pDNA transfer, for the most part because mRNA does not have to be transported into the nucleus. Consequently, early and dramatically higher protein expression has been reported.

Though the above listed advantages for the usage of mRNA for nonviral gene transfer should be emphasized, it must be noted that mRNA undergoes approximately 13 different nucleoside modifications including methylation in eukaryotic cells, and in vitro transcribed mRNA causes strong immune responses mediated by TLR3, TLR7 and TLR8, which represents a major challenge for its successful in vivo application. However, modified nucleosides may contribute to a reduction of these immune stimulatory effects, as shall be discussed later.

Mature mRNA in eukaryotic cells consists of five significant portions: the cap structure ([m7Gp3N (N: any nucleotide)], the 5'untranslated region (5'UTR), an open reading frame (ORF), the 3'untranslated region (3'UTR), and a tail of 100-250 adenosine residues (Poly(A) tail). In vitro transcribed mRNA can be obtained from plasmid DNA harboring a bacteriophage promoter, such as T7, SP6, or T3. In vitro transcription is a common technique using commercially available kits to obtain sufficient amounts of functional mRNA. Thus far, feasibility and technical refinement have been dramatically improved.

It was found that one-third to one-half of the caps are incorporated in the reverse orientation during in vitro transcription, making them unrecognizable to the cap-binding protein, eukaryotic initiation factor 4E (eIF4E). Instead of the normal cap structure, it was discovered that an anti-reverse cap analog (ARCA), m₂^{7,3'O}Gp₃G and m⁷3'dGp₃G in which a 3'OH group of a normal cap is removed or replaced with OCH₃ could avoid the cap incorporation in the wrong orientation. Subsequently, a high number of modifications on ARCA have been reported. Intriguingly, it was found that modifications not only at the C3' position, but also at the C2' position prevent reverse incorporation. Moreover, tetraphosphate ARCAs can promote translation more efficiently than other cap analogs. As a result, in vitro ARCA-capped transcripts (ARCA-mRNA) showed significantly higher translation efficiency compared to normal capped transcripts (CAP-mRNA) in a rabbit reticulocyte lysate. Further, it has been reported that m₂^{7,3'O}Gpp_{CH2}pG or m₂^{7,3'O}Gp_{CH2}ppG, in which the bridging oxygen in the α-β-linkage or β-γ-linkage, was substituted by a methylene group, respectively, were found to be resistant to hydrolysis by human Dcp2, one of the decapping enzymes, in vitro, and increased mRNA stability (Grudzien et al., 2006. J Biol Chem, 281, 1857-67). However, m₂^{7,3'O}Gpp_{CH2}pG showed only 52-68% affinity for eIF4E compared to m₂^{7,3'O}Gp₃G. It was recently reported that a phosphorothioate on ARCA (S-ARCA) stabilized and increased the efficiency of translation. It was found that luciferase (luc) mRNA capped with a sulphur substitution for a nonbridging oxygen in the β-phosphate moiety on ARCA, m₂^{7,2'O}GppₛpG (D2), was translated 5.1-fold more efficiently than a normal cap. Another diastereoisomeric form (D1) showed 2.8-fold higher translation efficiency. There was no significant difference in the efficiency of translation between S-ARCA and ARCA. However, t_{1/2} in D2 (257 min) was found to be strongly prolonged compared to normal cap (86 min) or ARCA (155 min). It seems therefore that the phosphorothioate contributes to the resistance to hydrolysis.

The poly(A) tail plays an important role in both mRNA translation and stability. The poly(A) tail binds to polyadenosyl binding protein (PABP). PABP interacts with the N-terminus of eIF4G, which leads to mRNA circularization. In addition, the poly(A) tail is able to bind numerous PABPs, whose interaction with eIF4G results in an increase for the affinity of eIF4E to the cap structure. The Cap-poly(A) interaction cooperatively results from the physical interactions between mRNA 5'and 3'ends. Once the poly(A) tail is removed or shortened to less than 12 residues, degradation of mRNA occurs through the cleavage of the 5'cap structure and 5' to 3'exonucleotidic digestion or 3' to 5'degradation. These observations illustrate that the poly(A) tail is very important to inhibit decapping as well as degradation of mRNA. For in vitro transcription, unless the template plasmid DNA contains a poly(d(A/T) tail, it may be post-polyadenylated by the poly(A) polymerase. However, in this case the length of the poly(A) tail may vary from reaction to reaction and within one approach, although this variation remains surprisingly low.

Intriguingly, it has been reported that although capped polyadenylated mRNA translation was inhibited by the addition of exogenous poly(A) in trans, the translation of capped nonpolyadenylated mRNA was rather stimulated under certain poly(A) concentrations. However, the addition of exogenous poly(A) which consists of 10-180 residues in trans in rabbit reticulocyte lysates stimulated translation of capped mRNA with a 100 adenosine residue poly(A) tail 11-fold. The addition of a poly(A) tail in the range of 15-600 residues resulted in a 2.3-fold stimulation of protein expression by co-transfection of ARCA-luc mRNA-A100 using lipofection.

Besides, both the cap structure and the poly(A) tail have been reported to individually contribute to the level of protein expression. ARCA-luc mRNA-A64 or 100 showed 25-fold, and 50-fold higher luciferase activity than CAP-luc mRNA-A64 or 100, respectively, using lipofection in mouse dendritic cells (JAWSII). In addition, ARCA-luc mRNA-A100 showed 700-fold higher luciferase activity than CAP-luc mRNA-A64. Hence, a long poly(A) tail combined with a modified cap structure, ARCA, greatly improves expression efficiency in dendritic cells.

It lies in the nature of enzymatic reactions that luciferase activity only indirectly measures protein expression levels, which means that the actual effects on translation efficiency remain to be determined. It was examined whether the length of the poly(A) tail (A0, A20, A40, A60, A80 and A100) affects expression levels not only in dendritic cells but also in other cells types. Interestingly, it was found that the translation efficiency increased using a poly(A) tail with a length up to A60, then declined with increasing poly(A) tail length in UMR-106, an osteoblast-like osteosarcoma cell line from rat. The effect of length of poly(A) on translation, therefore, might be cell type-dependent.

Also the impact of mRNA modifications on its stability and translational efficiency in dendritic cells was investigated. Various important factors were discovered to increase the stability and translational efficiency of mRNA by i) extending the length of poly(A) to A120; ii) the use of type IIS restriction enzymes such as SapI and BpiI to avoid an overhang at the 3' end of the poly(A) tail and to obtain a free-ending poly(A) tail when performing the linearization of the template plasmid vector; iii) two sequential 3'UTRs of the human β-globin gene cloned in between ORF and the poly(A) tail.

A variety of transfection reagents have been evaluated for their ability to deliver mRNA. While until now most publications suggest lipoplexes for mRNA transfection, polyplexes based on polyethylenimine (PEI, 25 and 22 kDa) led to rather poor results. The use of polycations however has been only rarely described in literature, though DEAE-dextran, poly(L-lysine) and dendrimers were capable of transfecting mRNA into cells in vitro.

The feasibility of mRNA transfer in mammalian cells utilizing cationic lipids has been already described in the late 1980's. DOTMA, a synthetic cationic lipid, incorporated into a liposome (lipofectin) was used to efficiently transfect mRNA into different cell lines in vitro. Different amounts of applied mRNA yielded a linear response of luciferase activity. Currently, DOTAP seems to be the most efficient and the most widely used cationic lipid, relatively cheap and efficient in both in vitro and in vivo mRNA delivery applications. In addition, cationic polymers may be used for mRNA transfection. Certain synthetic vectors based on reducible polycations exceeded the mRNA transfection efficiencies of the 25kDa PEI by far. However, if those modified vectors can be used directly for in vivo gene transfer remains to be investigated. With respect to the transfection mechanisms it was found out that the binding strength between the cationic polymer or lipid represented one of the critical parameters which affected mRNA expression efficiency. Whereas cationic polymers such as branched PEI 25 kDa and linear PEI 22 kDa, which were effective for plasmid DNA delivery and tightly bound to mRNA, did not result in detectable expression, low molecular weight PEI 2 kDa bound mRNA less efficiently but led to high expression levels in the presence of endosomolytic agents such as chloroquin or chemically linked melittin comparable to DOTAP. These observations demonstrate that single stranded mRNA binding to cationic polymers is stronger than pDNA binding. It was suggested that cytosolic RNA is involved in pDNA release from the cationic polymer as a precondition for nuclear entry and transcription. Therefore, the design of novel cationic polymers for mRNA delivery has to carefully address nucleic acid binding strength and efficient cationic polymers used for pDNA delivery may not be suitable for mRNA delivery.

Apart from polymeric and liposomal vector systems, an interesting further option that became prominent during the last years is the use of electroporation. Protocols for the delivery of exogenous RNA have been developed, resulting in 50-90% transfection efficiency in human hematopoietic cells and human embryonic stem cells. As the mRNA does not have to enter the nucleus, soft electrical pulses may be applied, reducing cell toxicity. Another advantage of electroporation might be that the RNA is shuttled directly into the cytosol, therefore possibly not being sensed by innate RNA receptors, which could surpass unwanted immune responses.

It has already been shown that in vivo application of bacterial DNA may lead to strong immune responses, especially via unmethylated CpG motifs. In contrast to naked DNA, which induces only a mild cytokine response, its complexes with cationic lipids lead to a strong cytokine response). Whereas varying the administration routes of cationic lipids did not markedly alter inflammatory cytokine expression, PEI-DNA either after intravenous injection or aerosol delivery resulted in lower lung cytokine levels compared to cationic lipids presumably due to their different endosomal uptake and thus interaction with the TLR9 receptor.

Responses to RNA delivery are much less explored. Both DNA and RNA stimulate the mammalian innate immune system through activation of Toll-like receptors (TLRs). Thirteen TLRs (named simply TLR1 to TLR13) have been identified in humans and mice together, and equivalent forms of many of these have been found in other mammalian species. Remarkably, different TLRs can recognize several structurally unrelated ligands. The TLR-mediated innate immune system has a bow-tie architecture in which a variety of pathogens and their molecules are represented by a much smaller number of ligands. The subcellular localization of different TLRs correlates to some extent with the molecular patterns of their ligands. Hence, TLR3, TLR7, TLR8 and TLR9 - all of which are involved in the recognition of nucleic-acid like structures, are localized intracellularly. TLR3 recognizes dsRNA, siRNA and mRNA, while TLR7 and TLR8 bind ssRNA and the recognition of CpG DNA motifs is mediated via TLR9.

In line with DNA CpG methylation (that suppresses recognition via TLR9), the immunogenicity of RNA seems to be under the control of similar types of modification. In vitro transcribed RNA resulted in strong TNF-alpha response by dendritic cells if they showed no mammalian-typical modifications. Intriguingly, the modification of specific nucleotides (e.g. N6-methyladenosine or pseudouridine) reduced the TLR3, TLR7 and TLR8 mediated cytokine secretion and activation of DCs dramatically. Hence, it may be possible to eliminate exaggerated in vivo immune responses by introducing modified NTPs into the in vitro transcription reaction. Replacement of only 25% of uridine and cytidine with 2-thiouridine and 5-methyl-cytidine synergistically decreased mRNA binding to pattern recognition receptors, such as TLR3, TLR7, TLR8 and RIG-I, in human peripheral blood mononuclear cells (PBMCs). These modifications substantially decreased activation of the innate immune system in vitro and in vivo and concomitantly increased the stability of the mRNA, allowing for prolonged, high-level cellular protein expression in >80% of cultured human and mouse alveolar type II epithelial cells as demonstrated by flow cytometry and cytokine ELISAs of cell culture supernatants and mouse blood sera. Overcoming intrinsic mRNA immunogenicity is considered to be critical to enable novel therapies which require repeated dosing for instance for the treatment of inherited and metabolic diseases or in the field of regenerative medicine.

In contrast to the foregoing, the strong immunostimulatory effect of RNA is used for therapeutic vaccination. Especially dendritic cells (DCs) as antigen presenting cells (APCs) are targeted by vaccine immunogens, which is followed by an activation of antigen-specific T and B cells. Several in vivo and in vitro studies have shown that targeting DCs with mRNA induced tumor immunity or anti-tumor responses. Compared to transfection of pDNA, mRNA based gene transfer led to higher tumor antigen loading of DCs and had a higher potential to stimulate cytotoxic T lymphocyte responses. Apart from anti-tumor approaches the ambition arised to use RNA-transfected DCs to cure or prevent infectious diseases like AIDS, hepatitis C or fungal infection. Another elegant strategy to achieve RNA vaccination is to express a target antigen by a bi-cistronic replicative RNA which codes both for the antigen and a RNA replicase, thereby utilizing the ability of alphaviruses to produce large amounts of viral mRNA. If a cell is transfected, the viral RNA is amplified by the replicase complex which synthesizes a genomic negative-strand that itself represents the template for the synthesis of many genomic RNA positive-strands by the RNA replicase. This approach has already been used in a mouse model to break tolerance and provide immunity to melanoma.

The present invention has now enabled a suitable way of efficiently delivering mRNA to lung cells and allowing effective expression of the protein encoded by the mRNA in these cells.

The invention is explained in more detail in the following example and the attached figures, yet without being restricted thereto.

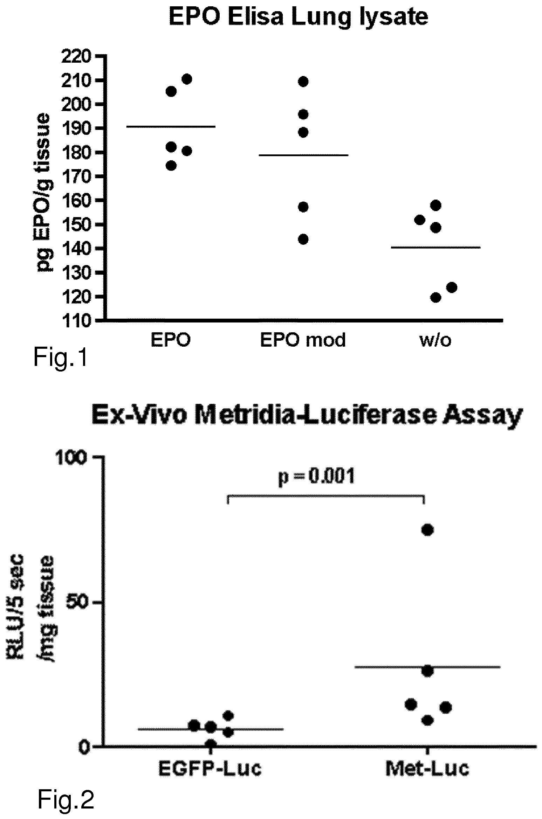

Fig. 1 shows the results of EPO expression measured by ELISA in lung lysate of mice 24 hours after aerosol treatment with a combination comprising the EPO mRNA and PEI 25 kDa (indicated EPO) or the chemically modified EPO mRNA and PEI 25 kDa (indicated EPO mod) in comparison with untreated mice (w/o). EPO levels are significantly increased for both treatment groups when compared to untreated mice.
Fig. 2 shows the results of the Metridia-Luciferase expression measured by luminescence activity in lung lysate of mice 24 hours after aerosol treatment with a combination comprising chemically modified MetLuc mRNA and PEI 25 kDa (indicated Met-Luc) or comprising chemically modified EGFPLuc mRNA and PEI 25 kDa (indicated EGFP-Luc) which serves as control. Metridia-Luciferase levels are significantly increased for group of mice treated with chemically modified MetLuc mRNA/PEI 25 kDA when compared to control mice treated with chemically modified EGFPLuc mRNA/PEI 25 kDA.
Fig. 3 shows that chemically modified Luc mRNA is effectively expressed in the lung cells of the mice upon pulmonary aerosol delivery as a combination with PEI 25 kDa (Fig. 3a+b).
Fig. 4 shows that luciferase expression is highest for chemically modified Luc mRNA comprising a cap-1.
Fig. 5 shows that chemically modified Luc mRNA is effectively expressed in the lung cells of a pig upon pulmonary aerosol delivery as a combination with PEI 25 kDa (Fig. 5B), whereas no Luc expression is seen in lungs of control animals treated with nebulized water (Fig. 5A).
Fig. 6 shows that water for injection (WFI) stabilizes chemically modified mRNA in PEI formulations; lanes:
   1 - modified mRNA in Aqua + Heparin
   2 - modified mRNA in PBS + Heparin
   3 - brPEI 25kDa/modified mRNA pH 7.4 in PBS/Aqua + Heparin (Method according to Densmore et al. EP 1 173 224 B1)
   4 - brPEI 25kDa/modified mRNA pH 7.4 in PBS + Heparin (Method Ethris)
   5 - brPEI 25kDa/modified mRNA pH 7.4 in Aqua + Heparin
   6 - brPEI 25kDaI/modified mRNA pH 6.0 in Aqua + Heparin
   7 - brPEI 25kDa/modified mRNA pH 5.0 in Aqua + Heparin.

### EXAMPLES

### 1. In vivo aerosol application of chemically modified and unmodified mRNA encoding erythropoietin (EPO) and Metridia luciferase (metLuc) formulated with polyethylenimine (PEI) to the lungs of mice

### Chemicals

Branched PEI (average MW = 25 kDa) was obtained from Sigma-Aldrich (Schnelldorf, Germany) and used without further purification. PEI was diluted in double-distilled water and adjusted to pH 7 with HCl. Double distilled endotoxin free water was purchased from Delta Pharma (Boehringer Ingelheim, Germany).

### mRNA production

### Cloning of murine EPO (mEPO) cDNA into pVAXA120 vector

cDNA coding for mEPO was excised from pCR4EPO plasmid (purchased from Open Biosystems, catalog number MMM1013-99829153) via *EcoRI* digestion and cloned into the respective site of pVAXA120. Clones were screened for insert using PmeI digestion and for orientation using *NheI* (single digest) and *SmaI-XbaI* (double digest). Clones which were correct with all the three digests were used for RNA production.

### Production of mEPO mRNA

To generate template for *in vitro* transcription, plasmid was linearized downstream of the poly (A) tail via overnight digestion with XbaI (Fermentas) at 37 degrees °C and purified using chloroform extraction and sodium acetate precipitation as described by Sambrook et al. (Sambrook, J., Fritsch, E. F., and Maniatis, T (1989). In Molecular Cloning: A Laboratory Manual. Cold Spring Harbor Laboratory Press, N.Y. Vol 1, 2, 3). Complete linearization of plasmid template was confirmed on 1% agarose gel.

*In vitro* transcription of pVAXA120-mEPO was carried out with RiboMAX Large Scale RNA Production System - T7 (Promega, Germany) at 30 and 37 degrees C following manufacturer's protocol using the anti-reverse cap analogue (ARCA; P1-(5'-(3'-o-methyl)-7-methyl-guanosyl)P3-(5'-(guanosyl))triphosphate, sodium salt, Jena Biosciences, Germany). For *in vitro* transcription of chemically modified mEPO mRNA (EPO Mod) 25% of both Cytidine-5'-Triphosphate and Uridine-5'-Triphosphate were replaced by 5-Methylcytidine-5'-Triphosphate (TriLink, USA) and 2-Thiouridine-5'-Triphosphate (TriLink, USA). Purification of mRNA was performed by chloroform extraction and size exclusion chromatography on PD-10 columns (GE Healthcare, Germany). The produced mRNA was screened for activity by transfection of a bronchial epithelial cell line (BEAS-2B) and a human embryonic epithelial kidney cellline (HEK 293) and measurement of mEPO amounts by ELISA (R&D Systems, Germany). Significantly higher amounts of mEPO could be quantified from BEAS-2B transfected with mEPO mRNA produced at 30 degrees °C compared to its counterpart produced at 37 degrees °C.

### Cloning of Metridia Luciferase (MetLuc) ORF into pVAXA120 vector

ORF coding for MetLuc (Clonetech sequence) was synthesized and cloned into the *BamHI* / *EcoRI* sites of pVAXA120 by GeneArt AG (Germany). The received pVAXA120-MetLuc plasmid was further used for *in vitro* transcription.

### Production of chemically modified MetLuc mRNA

To generate template for *in vitro* transcription, plasmid was linearized downstream of the poly (A) tail via overnight digestion with *Xba*I (Fermentas) at 37 degrees C and purified using chloroform extraction and sodium acetate precipitation as described by Sambrook et al. (Sambrook, J., Fritsch, E. F., and Maniatis, T (1989). In Molecular Cloning: A Laboratory Manual. Cold Spring Harbor Laboratory Press, N.Y. Vol 1, 2, 3). Complete linearization of plasmid template was confirmed on 1% agarose gel.

*In vitro* transcription of pVAXA120-MetLuc was carried out with RiboMAX Large Scale RNA Production System - T7 (Promega, Germany) at 30 degrees C following manufacturer's protocol using the anti-reverse cap analogue (ARCA; P1-(5'-(3'-o-methyl)-7-methyl-guanosyl)P3-(5'-(guanosyl))triphosphate, sodium salt, Jena Biosciences, Germany). For *in vitro* transcription of chemically modified MetLuc mRNA 25% of both Cytidine-5'-Triphosphate and Uridine-5'-Triphosphate were replaced by 5-Methylcytidine-5'-Triphosphate (TriLink, USA) and 2-Thiouridine-5'-Triphosphate (TriLink, USA). Purification of mRNA was performed by chloroform extraction and size exclusion chromatography on PD-10 columns (GE Healthcare, Germany). The produced mRNA was screened for activity by transfection of a murine fibroblast cell line (NIH-3T3) and measurement of MetLuc-Activity using a Metridia Luciferase reporter assay.

### Animals

Six to eight week-old female BALB/c mice were obtained from Janvier, Route Des Chênes SecsBP5, F-53940 Le Genest St. Isle, France, and maintained under specific pathogen-free conditions. Mice were acclimatized to the environment of the animal facility for at least seven days prior to the experiments. All animal procedures were approved and controlled by the local ethics committee and carried out according to the guidelines of the German law of protection of animal life.

### Preparation of PEI-mRNA polyplexes

Polyplexes were formulated as follows: mRNA and PEI were diluted in 4.0 ml of double distilled water resulting in concentrations of 250 µg/ml mRNA and 326.3 µg/ml PEI, respectively (corresponding to an N/P ratio of 10). The mRNA solution was pipetted to the PEI solution, mixed by pipetting up and down, to yield a final mRNA concentration of 125 µg/ml. The complexes were incubated for 20 min at ambient temperature before use. It was observed within the course of the present invention that it is specifically advantageous to use only water without any buffers for complexation because otherwise nanoparticles may aggregate or be ineffective in mouse lungs (Rudolph et al., J. Mol Ther. 2005, 12: 493-501)

### Design of the aerosol devices

For the nebulization procedure in a whole body device, mice are placed in a 9.8 × 13.2 × 21.5 cm plastic box which can be sealed with a lid. At one narrow side of the box, four small holes are positioned as aerosol outflow. Through a whole at the opposite narrow side, the box is connected via a 2.1 cm diameter connecting piece to a 15.4 cm wide × 41.5 cm long plastic cylinder. The bottom of the cylinder is evenly covered with 150 g of silica gel (1-3 mm, #85330; Fluka, Switzerland) for drying the aerosol which is produced by a jet nebulizer jet nebulizer (PARI BOY® LC plus, PARI GmbH) connected to the other end of the cylinder. (Details described in Rudolph et al., J Gene Med. 2005, 7: 59-66).

### Measurement of EPO and MetLuc activity in lung homogenates

Twenty-four hours post administration mice were anaesthetized by intraperitoneal injection of medetomidine (11.5 µg/kg BW), midazolame (115 µg/kg BW) and fentanyl (1.15 µg/kg BW) and the peritonea were opened by midline incisions. After opening the peritonea by midline incisions, lungs were dissected from animals and perfused with PBS. Lungs were snap-frozen in liquid nitrogen and homogenized in the frozen state with mortar and pestle. After addition of 400 µl of lysis buffer containing 25 mM Tris pH 7.4, 0.1% Triton X-100 and Complete Protease Inhibitor (Roche Diagnostics GmbH, Penzberg, Germany), samples were incubated for 20 min on ice. The protein lysates were subsequently centrifuged at 10,000 rcf, 5 min. EPO activity in the supernatant was measured by ELISA (R&D Biosystems) and MetLuc activity was analyzed by measurement luminescence activity upon addition of coelenterazine as described by Hönig et al., Biomacromolecules 2010, 11: 1802-1809).

### Results:

The first experiment shows that both unmodified and chemically modified EPO mRNA is effectively expressed in the lung cells of the animals upon pulmonary aerosol delivery as a combination with PEI 25 kDa. This shows that the method for delivery into the lungs is independent from the chemical composition of mRNA. The second experiment shows that Metridia luciferase is effectively expressed in the lung cells of the animals upon pulmonary aerosol delivery as a combination with PEI 25 kDa. This shows that the method for delivery into the lungs is not restricted to a single coding mRNA but independent from the sequence the mRNA codes for. Together this shows that the object of the present invention can be properly addressed by the method and pharmaceutical preparations according to the present invention.

### 2. In vivo aerosol application of chemically modified mRNA encoding firefly luciferase (Luc) formulated with polyethylenimine (PEI) to the lungs of mice

### Chemicals

Branched PEI (average MW = 25 kDa) was obtained from Sigma-Aldrich (Schnelldorf, Germany) and used without further purification. PEI was diluted in water for injection and adjusted to pH 7.4 with HCl. Endotoxin free water was purchased from B.Braun (Melsungen, Germany).

### Production of chemically modified Luc mRNA

To generate template for the in-vitro-transcription (IVT) the plasmid pVAXA120-Luc was linearized by restriction digestion with NotI. Template was further purified by Chloroform-Ethanol-Precipitation. Quality of template was determined by native agarosegel electrophoresis. IVT was carried out with a standard IVT mix containing ribonucleotide triphosphates, an anti-reverse cap analogue (ARCA, m^{7,3'-O}GpppG) and T7 RNA Polymerase. Modifications were introduced using 25% of 5-methyl-cytidine-5'-triphosphate and 25% of 2-thio-uridine-5'-triphosphate. ARCA was used to ensure incorporation of cap only in the desired orientation. To generate mRNA containing cap-0 or cap-1 structure using a post capping procedure, IVT was performed without any cap analogue resulting in mRNA containing a 5' terminal triphosphate. Capping was performed using the Vaccinia virus Capping Enzyme, rGTP and S-Adenosyl methionine (SAM) as a methyl donor to add a 7-methylguanylate cap-0 structure (m7GpppG) to the 5'end of the mRNA. To add a methyl group at the 2'-o position of the first nucleotide adjacent to the cap-0 structure at the 5'end of the mRNA resulting from the post-capping, an mRNA Cap 2'-o-Methyltransferase and SAM were used. This methylation resulted in a cap-1 structure (m7GpppGm) of the mRNA cap. Purification of mRNA was performed by ammonium acetate precipitation. Modified Luc RNA was resuspended in aqua ad injectabilia and quality control was performed using UV-measurement, native agarose gel electrophoresis and transfection in NIH3T3 cells.

### Animals

Six to eight week-old female BALB/c mice were obtained from Janvier, Route Des Chênes SecsBP5, F-53940 Le Genest St. Isle, France, and maintained under specific pathogen-free conditions. Mice were acclimatized to the environment of the animal facility for at least seven days prior to the experiments. All animal procedures were approved and controlled by the local ethics committee and carried out according to the guidelines of the German law of protection of animal life.

### Preparation of PEI-mRNA polyplexes

Polyplexes were formulated as follows: mRNA and PEI were diluted in 4.0 ml of double distilled water resulting in concentrations of 250 µg/ml mRNA and 326.3 µg/ml PEI, respectively (corresponding to an N/P ratio of 10). The mRNA solution was pipetted to the PEI solution, mixed by pipetting up and down, to yield a final mRNA concentration of 125 µg/ml. The complexes were incubated for 20 min at ambient temperature before use. It was observed within the course of the present invention that it is specifically advantageous to use only water without any buffers for complexation because otherwise nanoparticles may aggregate or be ineffective in mouse lungs (Rudolph et al., J. Mol Ther. 2005, 12: 493-501)

### Design of the aerosol devices

For the nebulization procedure in a whole body device, mice are placed in a 9.8 × 13.2 × 21.5 cm plastic box which can be sealed with a lid. At one narrow side of the box, four small holes are positioned as aerosol outflow. Through a whole at the opposite narrow side, the box is connected via a 2.1 cm diameter connecting piece to a 15.4 cm wide × 41.5 cm long plastic cylinder. The bottom of the cylinder is evenly covered with 150 g of silica gel (1-3 mm, #85330; Fluka, Switzerland) for drying the aerosol which is produced by a jet nebulizer (PARI BOY® LC plus, PARI GmbH) connected to the other end of the cylinder. (Details described in Rudolph et al., J Gene Med. 2005, 7: 59-66) .

### Measurement of Luc activity in mouse lungs using in vivo bioluminescent imaging

Twenty-four hours post administration mice were anaesthetized by intraperitoneal injection of medetomidine (11.5 µg/kg BW), midazolame (115 µg/kg BW) and fentanyl (1.15 µg/kg BW). D-luciferin substrate (3 mg/50 µl PBS per mouse) was applied via the intranasal route (Buckley SM, Howe SJ, Wong SP, Buning H, McIntosh J, et al. (2008) Luciferin detection after intra-nasal vector delivery is improved by intra-nasal rather than intra-peritoneal luciferin administration. Hum Gene Ther). Bioluminescence was measured 10 minutes later, using an IVIS 100 Imaging System (Xenogen, Alameda, USA) and the camera settings: field of view 10, f1 f-stop, high-resolution binning and exposure-time of 10 min. The signal was quantified and analyzed using the Living Image Software version 2.50 (Xenogen, Alameda, USA) .

### Results:

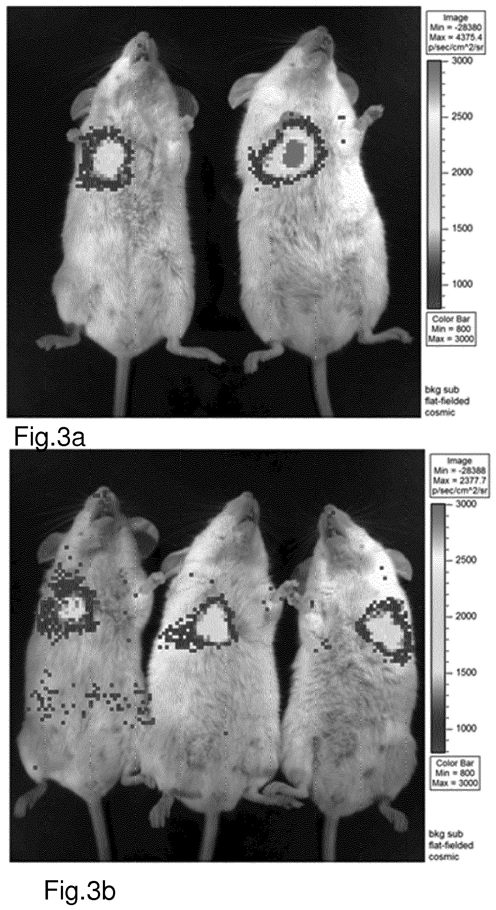

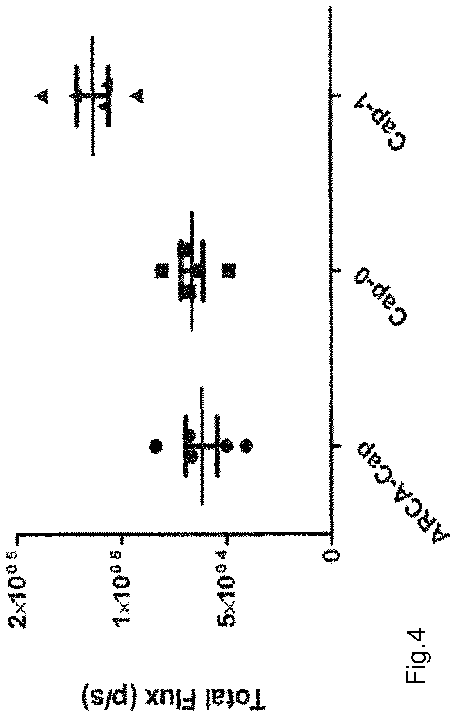

The experiment shows that chemically modified Luc mRNA is effectively expressed in the lung cells of the mice upon pulmonary aerosol delivery as a combination with PEI 25 kDa (Figure 3). Luciferase expression is highest for chemically modified Luc mRNA comprising a cap-1 (Figure 4). Together this shows that the object of the present invention can be properly addressed by the method and pharmaceutical preparations according to the present invention.

### 3. In vivo aerosol application of chemically modified mRNA encoding firefly luciferase (Luc) formulated with polyethylenimine (PEI) to the lungs of pig

### Chemicals

See example 2 above

### Production of chemically modified Luc mRNA

See example 2 above

### Experimental procedure

Sedation of the pig was initiated by premedication with azaperone 2 mg/kg body weight, ketamine 15 mg/kg body weight, atropine 0.1 mg/kg body weight and followed by insertion of an intravenous line to the lateral auricular vein. The pig was anesthetized by intravenous injection of propofol 3-5 mg/kg body weight as required. Anesthesia was maintained with continuous intravenous infusion of 1% propofol as required. Ventilation parameters were matched with endexpiratory carbon dioxide and adjusted if necessary. Anesthesia, respiratory and cardiovascular parameters were monitored continuously using pulse oximetry, capnography, rectal temperature probe and reflex status. The pig received infusion of balanced electrolyte solution at 10 ml/kg/h. Duration of the anesthesia was approximately 80-120 min. The pig was killed with bolus injection of pentobarbital 100 mg/kg of body weight via the lateral ear vein after sedation after aerosol application was completed (Aeroneb mesh nebulizer). Lungs were excised and sliced approximately 1 cm thick tissue specimens were collected from various lung regions followed by incubation in cell culture medium for 24 hrs at 37°C (5% carbon dioxide) in an incubator. For measurement of luciferase activity tissue specimens were incubated in a medium bath comprising D-Luciferin substrate in PBS (100 µg/ml) at 37°C for 30 min and subjected to ex vivo luciferase bioluminescent imaging (IVIS 100, Xenogen, Alameda, USA) .

### Preparation of PEI-mRNA polyplexes

Polyplexes were formed using a two channel syringe pump (KDS-210-CE, KD Scientific).mRNA and PEI were diluted each in 12.0 ml of double distilled water resulting in concentrations of 500 µg/ml mRNA and 650 µg/ml PEI, respectively (corresponding to an N/P ratio of 10). Both solutions were filled into a separate 20mL syringe using the withdrawal function of the syringe pump at a speed of 5mL/min. To mix both samples the two syringes were connected via a tubing (Safeflow Extension Set, B.Braun) was to a t-piece. Mixing was performed using the infusion function of the syringe pump at a speed of 40mL/min. The complexes were incubated for 30 min at ambient temperature before use. It was observed within the course of the present invention that it is specifically advantageous to use only water without any buffers for complexation because otherwise nanoparticles may aggregate or be ineffective in mouse lungs (Rudolph et al., J. Mol Ther. 2005, 12: 493-501).

### Results:

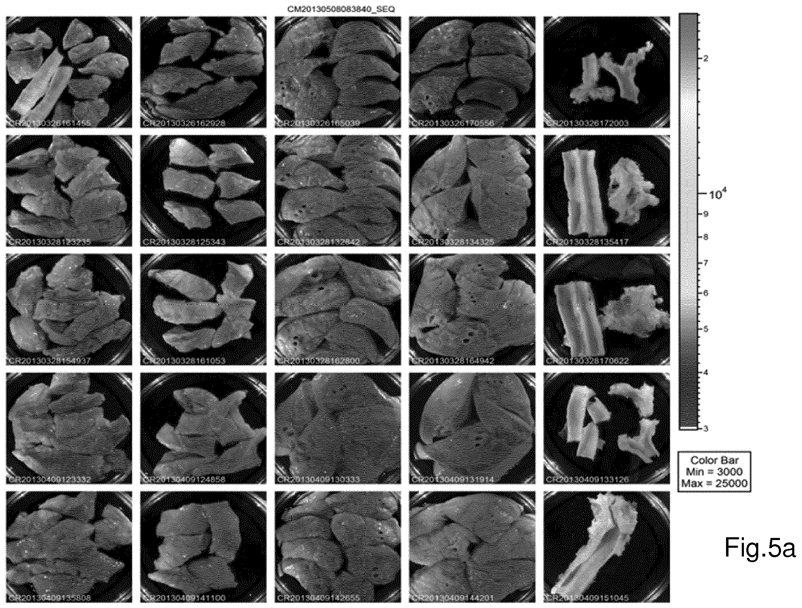

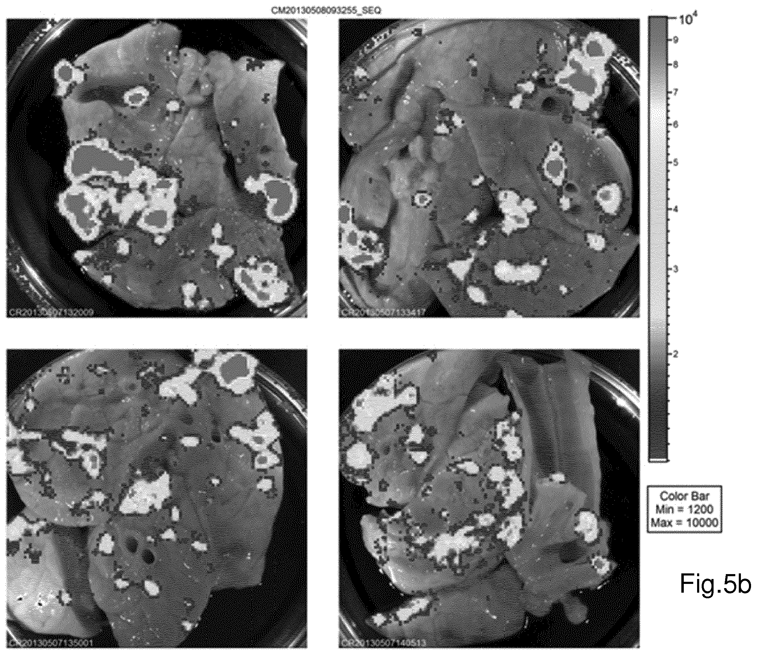

The experiment shows that chemically modified Luc mRNA is effectively expressed in the lung cells of a pig upon pulmonary aerosol delivery as a combination with PEI 25 kDa (Figure 5B), whereas no Luc expression is seen in lungs of control animals treated with nebulized water (Figure 5A). Together this shows that the object of the present invention can be properly addressed by the method and pharmaceutical preparations according to the present invention.

### 4. Water for injection (WFI) stabilizes chemically modified mRNA in PEI formulations

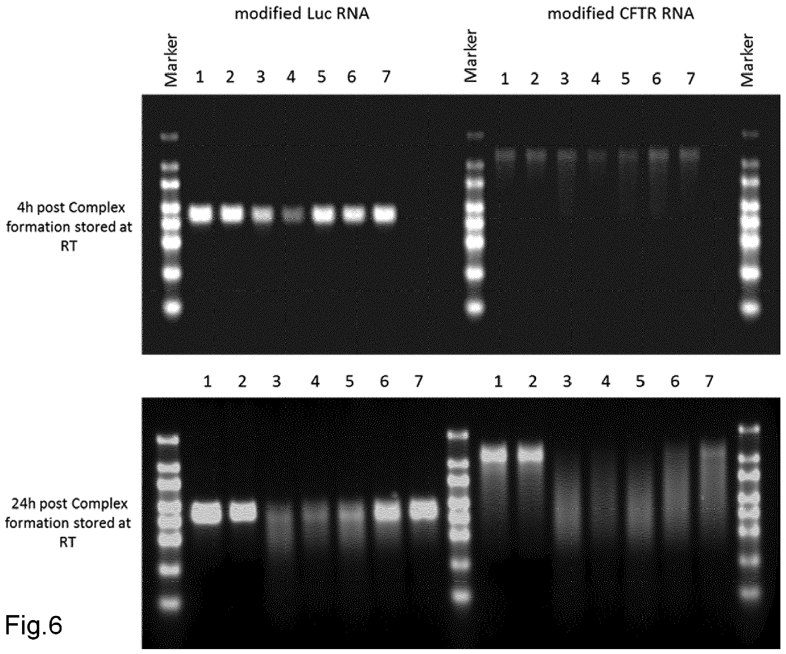

The effect of water for injection in comparison with PBS on mRNA stability has been examined by agarose gel electrophoresis (Figure 6). Whereas mRNA complexed with PEI in PBS leads to mRNA degradation as early as after 4 hrs of incubation at room temperature as indicated by a smear of degraded mRNA products, PEI/mRNA formulations in WFI are markedly stabilized as indicated by less mRNA degradation products (Figure 5) and greater band intensity of the main mRNA product. This effect is more pronounced for a large mRNA such as CFTR mRNA than for a shorter Luc mRNA and becomes even more evident after incubation at room temperature for 24 hrs. Importantly, mRNA degradation decreases with decreasing pH and reaches a minimum at pH=5. This observation is explains the most favorable properties and necessity of using WFI for mRNA aerosol delivery in PEI formulation because WFI is usually of acidic pH at values of pH=5 and therefore inherently stabilizes mRNA against degradation in aqueous PEI formulation.

### Experimental procedure

Preparation of mRNA-PEI polyplexes. Branched PEI 25kDa (Sigma-Aldrich, Schnelldorf) stock solutions were prepared at 10 mg/ml and 5 mg/ml in either Aqua ad Injectabilia (WFI, B.Braun, Melsungen) or Dulbecco's PBS (Life technologies, Darmstadt) and pH was adjusted with HCl to pH 7.4, pH 6.0 or pH 5.0.

25 µl modified mRNA (1 µg/µl) and 3.3 µl of PEI stock solution (10 mg/ml) were diluted in 50 µl WFI or D-PBS resulting in concentrations of 0.5 µg/µl mRNA and 0.66 µg/µl PEI, respectively (corresponding to an N/P ratio of 10). According to patent (EP 1173224 B1) 25 µl modified mRNA (1 µg/µl) and 6.6 µl of PEI stock solution in D-PBS (5 mg/ml) were diluted in 50 µl WFI. The PEI solution was slowly vortexed and the DNA solution was added to it to make a final volume of 100 µl. The mixture was allowed to stand at room temperature for 20 min before use.

For the Release-Assay using native Agarosegel electrophoresis 1 µl of polyplex solution was added to 4 µl of a heparin solution (40 mg/ml in WFI). The mixture was incubated at room temperature for 10 min. Following the incubation, 5 µl of 2X RNA Loading Dye (Thermo Fisher) was added and the samples were incubated at 70 degrees C for 10 min. Subsequently, the samples were placed on ice for 2 min and then loaded onto 1% agarose gel. The Gel was run at 180V for 1-1.5hrs and visualized using Intas Gel Documentation System.

The present invention relates to the following items:
1. Method for expressing an mRNA in lung wherein
   - the mRNA to be expressed is combined with polyethyleneimine (PEI) to provide a combination comprising the mRNA and PEI;
   - the combination comprising the mRNA and PEI is administered to lung where it enters lung cells; and
   - the mRNA is expressed in the lung cells.
2. Method according to item 1, wherein the lung is the lung of a human patient, preferably a human patient with a pulmonary defect, especially a pulmonary defect selected from the group surfactant protein B (SPB) deficiency, ATP-binding cassette sub-family A member 3 (ABCA3) deficiency, cystic fibrosis, alpha-1 antitrypsin (A1AT) deficiency, lung cancer, surfactant protein C (SPC) deficiency, alveolar proteinosis, sarcoidosis, acute and chronic bronchitis, emphysema, McLeod-Syndrom, chronic obstructive pulmonary disease (COPD), asthma bronchiale, bronchiectasis, pneumoconiosis, asbestosis, Acute Respiratory Distress Syndrome (ARDS), Infant respiratory distress syndrome (IRDS), pulmonary oedema, pulmonary eosinophilia, Löffler's pneumonia, Hamman-Rich syndrome, idiopathic pulmonary fibrosis, interstitial pulmonary diseases, primary ciliary dyskinesia, pulmonary arterial hypertension (PAH) and STAT5b deficiency, clotting defects, especially hemophilia A and B; complement defects, especially protein C deficiency, thrombotic thrombocytopenic purpura and congenital hemochromatosis, especially Hepcidin deficiency; pulmonary infectious diseases, preferably respiratory syncytial virus (RSV) infection, parainfluenza virus (PIV) infection, influenza virus infection, rhinoviruses infection, and severe acute respiratory syndrome (corona virus (SARS-CoV) infection, tuberculosis, Pseudomonas aeruginosa infection, Burkholderia cepacia infection, Methicillin-Resistant Staphylococcus aureus (MRSA) infection, and Haemophilus influenzae infection.
3. Method according to **item** 1 or 2, wherein the PEI has a molecular weight of 1 kDa to 1000 kDa, preferably from 10 kDa to 50 kDa, especially from 20 to 30 kDa.
4. Method according to any one of **items** 1 to 3, wherein the PEI comprises a targeting ligand, preferably a IP1 receptor ligand, more preferred a prostacyclin analogue, especially Iloprost (5-{ (E)-(1S,5S,6R,7R)-7-hydroxy-6[(E)-(3S, 4RS)-3-hydroxy-4-methyl-1-octen-6-inyl]-bi-cyclo[3.3.0]octan-3-ylidene}pentanoic acid) or Treprostinil ((1R,2R,3aS,9aS)-[[2,3,3a,4,9,9a-hexahydro-2-hydroxy-1-[(3S)-3-hydroxyoctyl]-1H-benz[f]inden-5-yl] oxy]acetic acid); or β2-aderonceptor ligands, especially Clenbuterol, Lactoferrin, uronic acids, or lectins.
5. Method according to any one of **items** 1 to 4, wherein the mRNA encodes Cystic fibrosis transmembrane conductance regulator (CFTR), Surfactant Protein B (SPB), ATP-binding cassette sub-family A member 3 (ABCA3) or alpha-1 antitrypsin (A1AT), surfactant protein C (SPC), erythropoietin, Factor VIII, Factor IX, van Willebrand Factor, granulocyte macrophage colony stimulating factor, ADAMTS 13, Hepcidin, angiotensin converting enzyme II or antigens of viral and bacterial pathogens.
6. Method according to any one of **items** 1 to 5, wherein the combination comprising the mRNA and PEI is administered to lung intratracheally, preferably as an aerosol, especially by spraying at high pressure.
7. Pharmaceutical composition comprising mRNA and PEI for use in a method for expressing the mRNA in lung.
8. Pharmaceutical composition according to **item** 7 for the treatment of a pulmonary defect, especially a pulmonary defect selected from the group surfactant protein B (SPB) deficiency, ATP-binding cassette sub-family A member 3 (ABCA3) deficiency, cystic fibrosis, alpha-1 antitrypsin (A1AT) deficiency, lung cancer, surfactant protein C (SPC) deficiency, alveolar proteinosis, sarcoidosis, acute and chronic bronchitis, emphysema, McLeod-Syndrom, chronic obstructive pulmonary disease (COPD), asthma bronchiale, bronchiectasis, pneumoconiosis, asbestosis, Acute Respiratory Distress Syndrome (ARDS), Infant respiratory distress syndrome (IRDS), pulmonary oedema, pulmonary eosinophilia, Löffler's pneumonia, Hamman-Rich syndrome, idiopathic pulmonary fibrosis, interstitial pulmonary diseases, primary ciliary dyskinesia, pulmonary arterial hypertension (PAH) and STAT5b deficiency, clotting defects, especially hemophilia A and B; complement defects, especially protein C deficiency, thrombotic thrombocytopenic purpura and congenital hemochromatosis, especially Hepcidin deficiency; pulmonary infectious diseases, preferably respiratory syncytial virus (RSV) infection, parainfluenza virus (PIV) infection, influenza virus infection, rhinoviruses infection, and severe acute respiratory syndrome (corona virus (SARS-CoV) infection, tuberculosis, Pseudomonas aeruginosa infection, Burkholderia cepacia infection, Methicillin-Resistant Staphylococcus aureus (MRSA) infection, and Haemophilus influenzae infection.
9. Pharmaceutical composition according to **item** 7 or 8, wherein the PEI has a molecular weight of 1 kDa to 1000 kDa, preferably from 10 kDa to 50 kDa, especially from 20 to 30 kDa.
10. Pharmaceutical composition according to any one of **items** 7 to 9, wherein the PEI comprises a targeting ligand, preferably a IP1 receptor ligand, more preferred a prostacyclin analogue, especially Iloprost (5-{ (E)-(1S,5S, 6R,7R)-7-hydroxy-6[(E)-(3S, 4RS)-3-hydroxy-4-methyl-1-octen-6-inyl]-bi-cyclo[3.3.0]octan-3-ylidene}pentanoic acid) or Treprostinil ((1R,2R,3aS,9aS)-[[2,3,3a,4,9,9a-hexahydro-2-hydroxy-1-[(3S)-3-hydroxyoctyl]-1H-benz[f]inden-5-yl] oxy]acetic acid); or β2-aderonceptor ligands, especially Clenbuterol, Lactoferrin, uronic acids, or lectins.
11. Pharmaceutical composition according to any one of **items** 7 to 10, wherein the mRNA encodes Cystic fibrosis transmembrane conductance regulator (CFTR), Surfactant Protein B (SPB), ATP-binding cassette sub-family A member 3 (ABCA3) or alpha-1 antitrypsin (A1AT), surfactant protein C (SPC), erythropoietin, Factor VIII, Factor IX, van Willebrand Factor, granulocyte macrophage colony stimulating factor, ADAMTS 13, Hepcidin, angiotensin converting enzyme II or antigens of viral and bacterial pathogens.
12. Pharmaceutical composition according to any one of **items** 7 to 11, further comprising at least one fluorocarbon.
13. Pharmaceutical composition according to any one of **items** 7 to 12, wherein the pharmaceutical composition is an aerosol.
14. Pharmaceutical preparation according to **item** 13, wherein the aerosol contains magnetic particles, especially magnetic particles with a diameter of at least 5 nm and at most 800 nm.
15. Pharmaceutical preparation according to **item** 14, wherein the magnetic particles have a diameter of at least 50 nm and at most 750 nm, preferably of at least 100 nm and at most 700 nm, more preferably of at least 150 nm and at most 600 nm, still more preferably of at least 200 nm and at most 500 nm, particularly preferably of at least 250 nm and at most 450 nm, most preferably of at least 300 nm and at most 400 nm.
16. Pharmaceutical composition according to any one of **items** 7 to 15, wherein the PEI is coupled to magnetic particles.
17. Pharmaceutical composition according to any one of **items** 14 to 16, wherein the magnetic particles consist of metals and/or oxides and/or hydroxides thereof or contain metals and/or oxides and/or hydroxides thereof.
18. Pharmaceutical composition according to **item** 17, wherein the metals are selected from the group consisting of iron, cobalt or nickel; and the oxides or hydroxides are selected from the group consisting of Fe₃O₄, gamma-Fe₂O₃, double oxides or hydroxides of di- or trivalent iron ions with Co²⁺, Mn²⁺, Cu²⁺, Ni²⁺, Cr³⁺, Gd³⁺, Dy³⁺ or Sm³⁺, and any mixtures thereof.
19. Pharmaceutical composition according to any one of **items** 14 to 18, wherein aerosol containing magnetic particles is deposited by a magnetic field onto the surface of the region of the respiratory tract and/or lung to be treated.
20. Pharmaceutical composition according to **item** 19, wherein the magnetic field has a field strength of at least 100 mT (millitesla), at least 200 mT, at least 500 mT or at least 1 T (tesla).
21. Pharmaceutical composition according to **item** 19 or 20, wherein the magnetic field has a magnetic field gradient of greater than 1 T/m or greater than 10 T/m.
22. Pharmaceutical composition according to any one of **items** 19 to 21, wherein the magnetic field is a pulsating, an oscillating or pulsating-oscillating magnetic field.
23. Pharmaceutical composition according to any one of **items** 19 to 22, wherein the magnetic field is matched dynamically to the breathing of the patient and is active only during the resting pauses between in- and exhalation of ex- and inhalation.
24. Pharmaceutical composition according to any one of **items** 7 to 23, wherein the composition has a pH of under 6.5, preferably of 3 to 6, especially of 4 to 5.5.
25. Pharmaceutical composition according to any one of **items** 7 to 24, wherein the composition has a 25°C conductivity of 10000 µS/cm or lower, preferably of 1000 µS/cm or lower, especially of 100 µS/cm or lower.

## Claims

1. Pharmaceutical composition for expressing an mRNA in lung, wherein said pharmaceutical composition comprises the mRNA to be expressed being combined with polyethyleneimine (PEI) to provide a combination comprising the mRNA and PEI, and wherein
- the combination comprising the mRNA and PEI is to be administered to lung where it enters lung cells; and
- the mRNA is to be expressed in the lung cells.

2. The pharmaceutical composition according to claim 1, wherein the lung is the lung of a human patient.

3. The pharmaceutical composition according to claim 2, wherein the lung is the lung of a human patient with a non-pulmonary defect or without a pulmonary defect.

4. The pharmaceutical composition according to any one of claims 2 or 3, wherein the lung of the human patient serves as a bioreactor for the secretion of a protein expressed by said mRNA from the lung cells into the blood circulation.

5. The pharmaceutical composition according to any one of claims 1 to 4, wherein the pharmaceutical composition or combination comprising the mRNA and PEI is to be administered to lung intratracheally, preferably as an aerosol, especially by spraying at high pressure.

6. The pharmaceutical composition according to any one of claims 1 to 5, wherein said pharmaceutical composition or said combination is to be administered to lung by inhalation via a nebulizer, is to be introduced into the respiratory tract via a nebulizer, is formulated for inhalation via a nebulizer and/or is formulated for introduction into the respiratory tract via a nebulizer.

7. The pharmaceutical composition according to any one of claims 1 to 6, wherein the mRNA to be expressed is complexed with

8. The pharmaceutical composition according to any one of claims 1 to 7, wherein PEI is coupled to magnetic particles.

9. The pharmaceutical composition according to any one of claims 1 to 8 for use in the treatment of a non-pulmonary defect, especially a non-pulmonary defect selected from the group consisting of McLeod-Syndrom and clotting defects, especially hemophilia A and B.

10. Pharmaceutical composition according to any one of claims 1 to 9, wherein the PEI has a molecular weight of 1 kDa to 1000 kDa, preferably from 10 kDa to 50 kDa, especially from 20 to 30 kDa.

11. Pharmaceutical composition according to any one of claims 1 to 10, wherein the PEI comprises a targeting ligand, preferably a IP1 receptor ligand, more preferred a prostacyclin analogue, especially Iloprost (5-{ (E)- (1S,5S,6R,7R)-7-hydroxy-6[(E)-(3S, 4RS)-3-hydroxy-4-methyl-1-octen-6-inyl]-bi-cyclo[3.3.0]octan-3-ylidene}pentanoic acid) or Treprostinil ((IR,2R, 3aS, 9aS)-[[2,3,3a,4,9,9a-hexahydro-2-hydroxy-1-[(3S)-3-hydroxyoctyl]-1H-benz[f]inden-5-yl]oxy]acetic acid); or β2-aderonceptor ligands, especially Clenbuterol, Lactoferrin, uronic acids, or lectins.

12. The pharmaceutical composition according to claim 11, wherein said targeting ligand is for target-specific delivering of a PEI-based pharmaceutical formulation to lung cells.

13. The pharmaceutical composition according to claim 12, wherein said target-specific delivering is to bronchial and/or alveolar epithelial cells.

14. The pharmaceutical composition according to any one of claims 1 to 10, wherein the pharmaceutical composition and combination does not comprise the targeting ligand as defined in anyone of claims 11 to 13.

15. The pharmaceutical composition according to any one of claims 1 to 10, wherein said combination or pharmaceutical composition consists of
(i) said mRNA and PEI;
(ii) said mRNA and PEI and
(A) distilled water;
(B) pharmaceutically acceptable water/water for injection (WFI);
(C) autoclaved ultrapure water; or
(D) tab water; or
(iii) (i) or (ii) and (a) pharmaceutically acceptable carrier(s) and/or (a) further auxiliary compound(s).

16. The pharmaceutical composition according to any one of claims 1 to 15, wherein the mRNA encodes erythropoietin, Factor VIII, Factor IX, van Willebrand Factor or ADAMTS 13.

17. The pharmaceutical composition according to any one of claims 1 to 16, wherein the pharmaceutical composition is an aerosol, preferably containing magnetic particles, especially magnetic particles with a diameter of at least 5 nm and at most 800 nm.

18. The pharmaceutical preparation according to claim 17, wherein the magnetic particles have a diameter of at least 50 nm and at most 750 nm, preferably of at least 100 nm and at most 700 nm, more preferably of at least 150 nm and at most 600 nm, still more preferably of at least 200 nm and at most 500 nm, particularly preferably of at least 250 nm and at most 450 nm, most preferably of at least 300 nm and at most 400 nm.

19. Pharmaceutical composition according to any one of claims 17 or 18, wherein the magnetic particles consist of metals and/or oxides and/or hydroxides thereof or contain metals and/or oxides and/or hydroxides thereof, preferably wherein the metals are selected from the group consisting of iron, cobalt or nickel; and the oxides or hydroxides are selected from the group consisting of Fe₃0₄, gamma-Fe₂03, double oxides or hydroxides of di- or trivalent iron ions with Co²⁺, Mn²⁺, Cu²⁺, Ni²⁺, Cr³⁺, Gd³⁺, Dy³⁺ or Sm³⁺, and any mixtures thereof.

20. Pharmaceutical composition according to any one of claims 1 to 19, wherein the composition has a pH of under 6.5, preferably of 3 to 6, especially of 4 to 5.5; and/or wherein the composition has a 25°C conductivity of 10000 pS/cm or lower, preferably of 1000 pS/cm or lower, especially of 100 pS/cm or lower.
